(19) Europäisches Patentamt
European Patent Office
Office européen des brevets

(11) Publication number: 0 217 115
B1

(12) # EUROPEAN PATENT SPECIFICATION

(45) Date of publication of the patent specification: **25.10.89**

(51) Int. Cl.⁴: **A61L 27/00**, A61F 2/06

(21) Application number: **86111688.7**

(22) Date of filing: **23.08.86**

(54) **Artificial vessel.**

(30) Priority: **26.08.85 JP 187137/85**
**12.09.85 JP 202411/85**

(43) Date of publication of application:
**08.04.87 Bulletin 87/15**

(45) Publication of the grant of the patent:
**25.10.89 Bulletin 89/43**

(84) Designated Contracting States:
**DE FR GB IT**

(56) References cited:
**DE-A- 3 204 719**
**GB-A- 1 265 246**
**US-A- 4 208 745**
**US-A- 4 286 341**
**US-A- 4 321 711**

(73) Proprietor: **KANEGAFUCHI KAGAKU KOGYO KABUSHIKI KAISHA, 2-4 Nakanoshima 3-chome, Kita-ku Osaka-shi Osaka-fu(JP)**

(72) Inventor: **Kira, Kazuaki, 1-12-33-1303, Ryugadai Suma-ku, Kobe-shi Hyogo-ken(JP)**

(74) Representative: **Türk, Gille, Hrabal, Bruckner Strasse 20, D-4000 Düsseldorf 13(DE)**

**EP 0 217 115 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to an artificial vessel having a porosity, a compliance approximate to that of a vital vessel, and a contact surface with blood having an excellent blood compatibility. More particularly, the present invention relates to an artificial vessel having a porosity, a compliance approximate to that of a vital vessel, and a contact surface with blood having an excellent blood compatibility, wherein the vessel wall is made of an elastomer having a porous structure and the vessel has a porosity percentage of from 95 to 75%.

In recent years, study on the artificial vessel has proceeded with a progress of vascular surgery and many artificial vessels have been developed. Hitherto, for an artificial vessel for artery of a medium- or large-caliber with a diameter of about not less than 6 mm, Debakey artificial vessel made of woven Dacron (R) (USCI. Co., Ltd. of U.S.A), Gore-Tex (R) (Gore Co., Ltd. of U.S.A.) which is made of an expanded polytetrafluoroethylene (hereinafter referred to as "EPTFE"), and the like have been clinically used.

The above artificial vessels have pores which communicate the inside and the outside of the vessel wall. Soon after the artificial vessel is grafted into a living body, the vessel is covered with pseudointima and the pseudointima grows into the communicating pores, which makes the artificial vessel encapsulated to serve as the artificial vessel. Such property of having the communicating pores suited for encapsulation is hereinafter referred to as "porosity".

However, these artificial vessels have a disavantage of a poor patency since they have a mechanical property, especially a compliance extremely different from that of a vital vessel and a contact surface with blood having a bad blood compatibility. From this reason, they cannot be clinically used as the artificial vessel for vein or in a vascular reconstructive surgery of artery of a small-caliber with an inner diameter of not more than about 6 mm and self-veins have hitherto been used in vascular reconstructive surgery of arteries below knees or of coronary artery.

US-S 4 321 711 describes a vascular prothesis having a porous elastomer coating, said coating having a porosity of 50 to 95%.

For obtaining the artificial vessel having an excellent patency, especially the artificial vessel suited for application in the vascular reconstructive surgery of artery of small-caliber, it appears to be important for the artificial vessel to have a mechanical property, especially a compliance approximate to that of a vital vessel and to have a contact surface with blood having an excellent blood compatibility as well as to have a porosity, which improves the patency of the artificial vessel. However, such artificial vessel has hitherto not yet been prepared.

Under the above-mentioned circumstances, the present invention is aimed at providing an artificial vessel with an excellent patency having a porosity, a mechanical property (especially a compliance) approximate to that of a vital vessel, and a contact surface with blood having an excellent blood compatibility, especially the artificial vessel which can also be used as an artificial vessel of small-caliber with an inner diameter of not more than about 6 mm.

According to the present invention, there is provided an artificial vessel, wherein the vessel wall is made of a thermoplastic elastomer having a porous structure and the vessel has a porosity percentage of from 95 to 75%. The present invention was made from the finding that the artificial vessel can be prepared with a porosity, a mechanical property (especially a compliance) approximate to that of a vital vessel and a contact surface with blood having an excellent blood compatibility, by controling a porosity percentage.

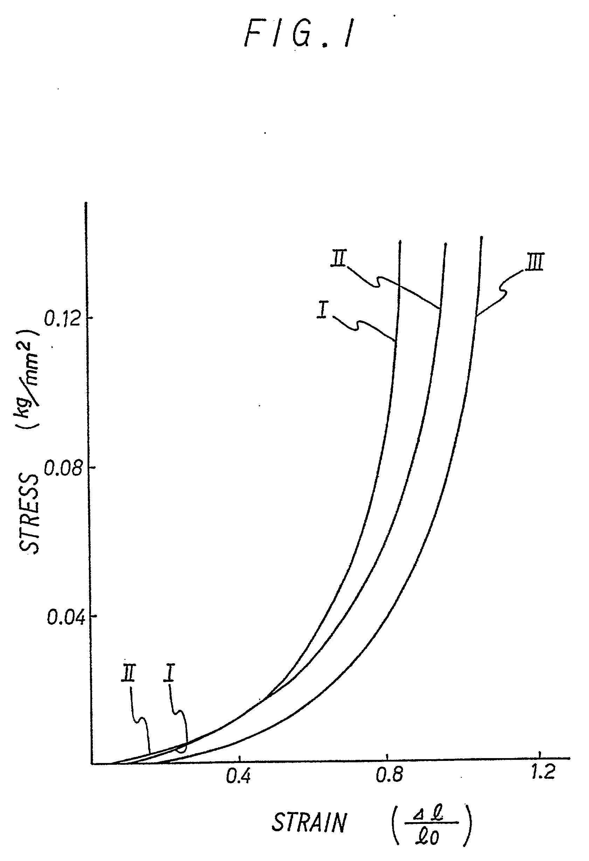

Fig. 1 is a graph of stress-strain curves of carotid artery (curve I), the artificial vessel prepared in Example 4 (curve II) and thoracic aorta (curve III), respectively.

The vessel wall of the artificial vessel of the present invention is made of a thermoplastic elastomer having a porous structure.

The porous structure of the thermoplastic elastomer contains pores which pass through the whole thickness of the vessel wall from the inner surface to the outer surface and has a porosity. The pores are communicated with each other in at least one part thereof and there are openings formed by at least one part of the pores at the inner surface and the outer surface of the vessel wall. A partition which forms the pores is made of the elastomer and connected with each other continuously. Preferably, the partition itself contains a large number of small pores or holes so that the vessel wall has a more bulky structure and the artificial vessel having a compliance approximate to that of a vital vessel is obtained.

More preferable porous structure is a network structure in which a pore number (a pore number per unit volume, hereinafter the same) at the thickness of at least 30 μm from the inner surface of the vessel wall is larger than that at the rest of the thickness of at least 30 mm from the inner surface of the vessel wall.

Preferably, the pores present over the entire thickness of the vessel wall from the inner surface to the outer surface have substantially uniform size.

Since the vicinity of the inner surface and of the outer surface of the vessel wall has a slightly more condensed structure than the rest which is between the vicinity of the inner surface and the vicinity of the outer surface and occupies a great portion of the porous structure, the pores are sometimes not uniform over the entire thickness of the vessel wall. However, if such ununiformity does not impair the porosity, the pores are estimated to be substantially uniform. Although a maximum diameter of a cross section of the pores is not particularly limited, it is preferably I to I00 μm, more preferably 3 to 75 μm. When tee maximum diameter is more than I00 μm, a strength of the vessel wall is decreased or the porosity becomes too large. When the maximum diameter is less than I μm, the porosity becomes poor or the compliance becomes too small.

Although a shape of the openings present at the inner surface and the outer surface of the porous structure is not particularly limited, a shape of the openings present at the inner surface is preferably a round or oval form. A maximum diameter of such shape is preferably from I to I00 μm, more preferably from 5 to 50 μm, and most preferably from I0 to 30 μm. When the maximum diameter is more than I00 μm, a blood flow is disturbed and an antithrombogenisity of the artificial vessel is lowered. When the maximum diameter is less than I μm, the encapsulation of the artificial vessel becomes poor.

The artificial vessel of the present invention has a porosity percentage of from 95 to 75 %. It is preferable that the porosity percentage at the thickness of at least 30 μm from the inner surface of the vessel wall is from 95 to 80 % and the porosity percentage at the rest of the thickness of at least 30 μm from the inner surface of the vessel wall is smaller than that at the thickness of at least 30 μm from the inner surface of the vessel wall. It is more preferable that the porosity percentage at the thickness of at least 50 μm from the inner surface of the vessel wall is from 95 to 83 % and the porosity percentage at the rest of the thickness of at least 50 μm from the inner surface is smaller than that at the thickness of at least 50 μm from the inner surface of the vessel wall, the artificial vessel having the porosity percentage of from 90 to 80 % as a whole.

The "porosity percentage" in the present invention is defined by the following equation (I).

$$\text{porosity percentage} = \frac{V - \frac{W}{A}}{V} \times 100 \qquad (1)$$

In the above equation (I), V is a vessel wall volume of the artificial vessel, W is a weight of the artificial vessel, and A is a specific gravity of the material forming the artificial vessel. The porosity percentage is determined by an amount of the pores present in the vessel wall and an amount of the small pores and holes present in the partition which forms the pores.

In the artificial vessel of the present invention, the porosity percentage greatly affects the mechanical property, especially the compliance, and the blood compatibility of the contact surface with blood. The porosity percentage has a positive correlation with the compliance, i.e. the compliance increases as the porosity percentage increases. The porosity percentage of more than 95 % is not preferable since at this porosity percentage the compliance of the artificial vessel becomes larger than that of a vital vessel.

The porosity percentage at the thickness of at least 30 μm from the inner surface of the artificial vessel is nearly in proportion to a number of the pores present at the inner surface, i.e. the contact surface with blood, of the artificial vessel. Further, the more the number of the pores present at the contact surface with blood increases, the more the patency of the artificial vessel is improved with the preferable encapsulation and the excellent blood compatibility. When the porosity percentage at the thickness of at least 30 μm from the inner surface of the artificial vessel is less than 75 %, the blood compatibility tends to become poor. Therefore, in order to control the mechanical property, especially the compliance, and the number of the pores present at the contact surface with blood by controlling the porosity percentage, it is advantageous that the number of the pores present at the contact surface with blood is increased by increasing the porosity percentage at the thickness of at least 30 μm from the inner surface of the vessel wall, and the mechanical property, especially the compliance, is controled by lowering the porosity percentage at the rest of the thickness of at least 30 μm from the inner surface of the vessel.

The elastomer used in the present invention is a thermoplastic elastomer having an excellent antithrombogenisity which does not release any low molecular compound, which causes acute poisoning, inflammation, hemolysis, fever and the like, and does not seriously impair the physiologic function of blood. Examples of such elastomers are, for instance, polystyrene elastomers, polyurethane elastomers, polyolefin elastomers, polyester elastomers, and the like. The above elastomers can be used in a single form or as a mixture thereof.

Since it is enough for the elastomer to have characteristics of the elastomer only when formed into the artificial vessel, even a mixture of the above elastomer with a polymer not having characteristics of the elastomer can be used as the elastomer in the present invention insofar as the final product has characteristics of the elastomer.

Among the above elastomers, a thermoplastic polyether type segmented polyurethane, including segmented polyurethane urea, hereinafter the same, elastomers are more preferable in viewpoint of strength, elongation, durability, antithrombogenisity, processability and the like. A segmented polyurethane containing fluorine atom in a hard segment or a soft segment, and a segmented polyurethane disclosed in Japanese Unexamined Patent Publication (KOKAI) No. 2II358/I982, which contains poly-

dimethylsiloxane in its main chain are still more preferable. Particularly preferable elastomers are a segmented polyurethane which contains, in a part of a soft segment, polydimethylsiloxane having the formula:

$$-R_1-(OR_2)_a-(CH_3)_b-(O)_c-(\overset{\overset{CH_3}{|}}{\underset{\underset{CH_3}{|}}{Si}}O)_d-\overset{\overset{CH_3}{|}}{\underset{\underset{CH_3}{|}}{Si}}-(O)_e-(R_4O)_f-(R_5O)_g-R_6-$$

wherein $R_1$, $R_2$, $R_3$, $R_4$, $R_5$ and $R_6$ are an alkylene group having at least 1 carbon atom, preferably an alkylene group having 2 to 6 carbon atoms such as ethylene, propylene, butylene or hexamethylene; a and g are 0 or an integer of 1 to 30, b, c, e and f are 0 or 1, and d is an integer of not less than 2, preferably from 5 to 135.

As mentioned above, the artificial vessel of the present invention has an excellent patency due to the porosity, the compliance approximate to that of a vital vessel, and the excellent blood compatibility of the contact surface with blood. However, in order to prevent a rupture or an impairment which may occur due to an abnormally high pressure such as in case of surgery, or to maintain a durability for a long period, the artificial vessel is preferably reinforced with tubular material made of fiber. Further, the artificial vessel is preferably reinforced with tubular material made of fiber so as to have a stress-strain curve approximate to that of a vital vessel.

Although the artificial vessel reinforced with tubular material made of fiber can be subjected to a sterilization procedure by gamma ray or ethylene oxide, it has a defect that the artificial vessel shrinks in a sterilization procedure by boiling or by high-pressure steam. Therefore, in order to obtain the artificial vessel which does not shrink even in the sterilization procedure by boiling or by high-pressure steam, the artificial vessel is preferably reinforced with heat-set tubular material made of fiber.

A "heat-set" procedure in the present invention is a procedure to heat the tubular material made of fiber to such a degree that the tubular material does not shrink in the sterilization procudure by boiling or by high-pressure steam, for example, at 121°C for 20 minutes. In practice, the heat-set procedure can be carried out by boiling, by exposing in steam, by maintaining a high temperature atmosphere, by conducting a sterilization by high-pressure steam, or the like. Among the above procedures, the heat-set procedure is preferably carried out by the sterilization by high-pressure steam, which allows to conduct the heat-set procedure surely and with a good operatability.

The heat-set tubular material made of fiber in the present invention may be any those prepared by heat-setting a fiber and then forming the fiber into a tubular material, by heat-setting a fiber, forming the fiber into a tubular material and further heat-setting the obtained tubular material, or by heat-setting a tubular material made of fiber itself. In viewpoint of operatability, the heat-set tubular material made of fiber is preferably prepared by heat-setting a tubular material made of fiber itself.

The artificial vessel reinforced with the heat-set tubular material made of fiber is an artificial vessel where at least a part of the tubular material is in contact with and/or is combined with the porous material made of elastomer, and a mechanical interaction exists between the tubular material and the porous material made of elastomer in such a degree that both the tubular material and the porous material show nearly the same strain against blood pressure or stress applied from the outside.

The fiber used in the present invention is a fine and long fiber having a length not less than 100 times larger than a diameter, which is usually employed for producing a yarn, a net yarn, a rope, a woven fabric, a knitting fabric, a braid, a nonwoven fabric, or the like. The fiber may be made of an organic material or of an inorganic material, insofar as the fiber does not give any bad influence to a living body, a degradation of the fiber in a living body is negligible, and the fiber is stable in the sterilization procedure, and also the fiber can be formed into the tubular material. From viewpoints of processability, commercial availability, pliablility and uniformity, there are preferably employed a regenerated man-made fiber, a semi-synthetic fiber and a synthetic fiber. Examples of the fiber are, for instance, cellulose type fibers, protein type fibers, polyamide type fibers, polyester type fibers, polyurethane type fibers, polyethylene type fibers, polystyrene type fibers, polyvinylchloride type fibers, polyvinylidene chloride type fibers, polyfluoroethylene type fibers, polyacrylic type fibers, polyvinyl alcohol type fibers, and the like. Among them, a fiber having a stretching property is more preferably employed. Examples of such a stretch fiber are, for instance, fibers having a self-stretching property such as rubber type fibers, polyurethane elastic type fibers or polyester elastic type fibers; fibers processed to have a stretching property such as stretch bulked processed fibers, covered yarns, fancy yarns, bulky yarns, A to Z yarns and conjugate fibers; and the like.

The tubular material made of fiber used in the present invention is a tubular material made of the above-mentioned fiber; a yarn spun from at least one of the above-mentioned fibers; a multifilament of at least one of the above-mentioned fibers; a woven fabric, a knitting fabric, a braid, a nonwoven fabric or a fabric combined thereof, which are produced from the above fiber, yarn or multifiber, and the like. A tubular material made of a polyurethane foam of sponge like structure can also be employed.

The tubular material made of fiber is preferably used so that the artificial vessel, when the tubular material and the porous structure are combined together, has a compliance approximate to that of a vital vessel and further a stress-strain curve approximate to that of a vital vessel. Such properties of the tubular material can be achieved by, for instance, either of the following two processes or a combination thereof. One process is to control the number of the connecting or contacting points of the fibers or yarns and to adjust the tightness of the connecting point of the fibers or yarns. Another process is to use a stretch fiber.

The tubular material may be formed by fiber or material made of fiber by itself or by combining the fiber with the porous material made of elastomer so that the tubular structure is formed at the finishing. In viewpoints of processability, workability and establishment of the stress-strain curve approximate to that of a vital vessel, there is preferably employed a tubular material made of knitting fabric of the fiber, more preferably a tubular material made of knitting fabric of stretch fiber.

In order to obtain the stress-strain curve approximate to that of a vital vessel, there are preferably employed, among the stretch fiber, stretch bulked processed fibers such as Woolie nylon and Woolie tetron, covered yarns which are prepared by winding another spun yarn or filament on the stretched rubber or spandex filament, and the like.

Although a stress-strain curve of a vital vessel cannot be sweepingly determined since it varies with a kind of a vessel such as artery or vein, a diameter of a vessel and the like, a vital vessel substantially has the stress-strain curve (I) or (III) as shown in Fig. I. The curves (I) and (III) are stress- strain curves of carotid artery and of thoracic aorta, respectively. These stress-strain curves show that an elastic modulus, which is low at a normal blood pressure level, increases drastically when a stress over the normal blood pressure level is applied. As shown in Fig. I, the stress-strain curve (II) of the artificial vessel of the present invention approximates to those of a vital vessel. The stress-strain curve can be measured with a tension testing machine usually employed in the polymer material field such as, for instance, Autograph AG-2000 A made by Shimazu Coorporation.

The "compliance" as used herein is defined by the equation (2):

$$C = \frac{\Delta V}{Vo \cdot \Delta P} \times 100 \qquad (2)$$

wherein C is a compliance, Vo is a volume of a measured vessel at the inner pressure of 50 mmHg, Δp is a pressure difference (l00 mmHg) from 50 mmHg to l50 mmHg of the inner pressure, ΔV is an increasing volume of the vessel when the inner pressure rises from 50 mmHg to l50 mmHg. In the practical measurement, a vessel is inserted into a closed circuit, and a volume of an injected liquid and a pressure variation in the circuit are measured by means of a microanalysis pump. From the results, the compliance can be calculated according to the above equation (2).

In case of the measurement of the artificial vessel having the porosity, the communicating pores in the vessel wall are plugged by a procedure such as pre-clotting.

A compliance of a vital vessel varies with a kind of a vital vessel such as artery or vein, a diameter of the vessel and the like. Therefore, although a preferable compliance for the artificial vessel cannot be sweepingly determined since it varies with a diameter of the artificial vessel, a region to which the artificial vessel is applied, and the like, the artificial vessel of the present invention is prepared so as to have the compliance approximate to that of each vital vessel, for example, the compliance of from 0.l to 0.8. For arteries of a small-caliber, the artificial vessel preferably has a compliance of from 0.l to 0.5.

A process for preparing the artificial vessel of the present invention is explained in the following description.

The artificial vessel of the present invention can be prepared by repeating one or two times a procedure of coating an elastomer solution containing a pore-forming agent and/or having a cloud point on a mandrel and then immersing the coated mandrel into a coagulating liquid. The porosity percentage can be controled by a particle size and an amount of the pore-forming agent, a solvent composition of the elastomer solution, a kind of the coagulating liquid and the like. In order to obtain the artificial vessel with the increased porosity percentage at the inner portion of the vessel wall, the above conditions are suitably controled at the procedure of preparing the inner portion of the vessel wall.

When the artificial vessel is reinforced with the tubular material made of fiber, the tubular material made of fiber is made to be present on the mandrel at any step of the above procedure.

The elastomer solution used in the present invention is classified into **(1)** an elastomer solution containing a pore-forming agent, **(2)** an elastomer solution having a cloud point and **(3)** an elastomer solution containing a pore-forming agent and having a cloud point.

The elastomer solution containing a pore-forming agent **(1)** essentially comprises a pore-forming agent, the elastomer and a solvent which can dissolve the elastomer (hereinafter referred to as "good solvent") in which the pore-forming agent is uniformly dispersed. The preferable amount of the pore-forming agent is preferably from l to 250 % (percentage of the volume of the pore-forming agent/the volume of the elastomer, hereinafter the same), more preferably from 20 to 200 %, and most preferably

from 50 to I50 %. When the elastomer solution is dipped into the coagulating liquid, the elastomer is deposited due to replacement of the good solvent with the coagulating liquid. The pore-forming agent in the deposited elastomer is dissolved and removed to give the porous structure of the artificial vessel of the present invention. If necessary for controlling a coagulation rate of the elastomer solution and a density or shape of the porous structure, a solvent which cannot dissolve the elastomer but can be miscible with the good solvent (hereinafter referred to as "poor solvent") may be added.

The elastomer solution having a cloud point **(2)** essentially comprises the elastomer, the good solvent and the poor solvent. The poor solvent is employed in such an amount that the solution has a cloud point. The "cloud point" means a temperature at which a dissolved polymer in a solution is deposited in a form of colloid, in other word, a temperature at which phase change occurs. When the elastomer solution **(2)** is handled at a temperature below the cloud point, it is difficult to form a uniform coating of the elastomer solution, and thus a proper porous structure cannot be obtained. Therefore, it is preferable to coat the mandrel with the elastomer solution at a temperature above the cloud point, and then immediately or after the phase change, to immerse the coated elastomer solution into the coagulating liquid of a temperature below the cloud point. According to the procedure, the porous structure can be formed by changing the phase of the elastomer solution in the coating layer and depositing the elastomer in the coagulating liquid in the above order or at the same time.

The elastomer solution containing a pore-forming agent and having a cloud point **(3)**essentially comprises the elastomer, the pore-forming agent, the good solvent and the poor solvent, the amount of the poor solvent being such an amount that the solution has a cloud point. From the elastomer solution **(3)**, the porous structure can be formed by depositing the elastomer and then dissolving to remove the pore-forming agent as in the case of the elastomer solution **(2)**.

The pore-forming agent used in the present invention is insoluble in the good solvent and can be removed from the prepared artificial vessel. The particle size of the pore-forming agent is preferably from I to I00 μm, more preferably from I0 to 74 μm, and most preferably from 20 to 50 μm. Examples of the pore-forming agent are, for instance, calcium carbonate, glucose, starch, casein, collagen, gelatin, albumin and the like.

The good solvent used in the present invention varies depending on a kind of the elastomer and is not particularly limited. Examples of the good solvent are, in case of the polyurethane elastomers, for instance, N,N-dimethylacetamide, N,N-dimethylformamide, N-methyl-2-pyrrolidone, dioxane, tetrahydrofuran, a mixture thereof, and the like.

As the poor solvent used in the present invention, there can be employed any solvent which cannot dissolve the elastomer, but can be miscible with the good solvent. Examples of the poor solvent are, for instance, water, lower alcohols, ethylene glycol, propylene glycol, I,4-butane diol, glycerine, a mixture thereof, and the like.

The coagulating liquid used in the present invention may be substantially the same as the poor solvent. Examples of the coagulating liquid are, for instance, water, lower alcohols, ethylene glycol, propylene glycol, I,4-butane diol, glycerin, a mixture thereof, and the like.

The mandrel used in the present invention is not particularly limited insofar as the mandrel is not dissolved in the elastomer solution. Preferable mandrel is a lod having a smooth surface such as a glass lod, a tefron lod or a stainless steel lod. When using dies having any shapes instead of the lod, various medical articles other than the above tubular article can be obtained. For instance, if a plate is used as the die, there can be provided a film-like article which can be utilized for an artificial skin.

The tubular material made of fiber is made to be present on the mandrel by, for instance, covering the mandrel with the tubular material made of fiber or by winding fiber or a strip made of fiber on the mandrel to form a tubular structure. The tubular material made of fiber may be made to be present on the mandrel directly, or on the mandrel on which the elastomer is deposited. It is preferable that the tubular material made of fiber is made to be present on the mandrel on which the elastomer is deposited, followed by repeating one or two times a procedure of coating the elastomer solution and of depositing the elastomer.

The artificial vessel prepared by the above procedures has the following excellent properties.

**(1)** The artificial vessel has a porosity useful for encapsulation of the vessel.
**(2)** The artificial vessel has the contact surface with blood having an excellent blood compatibility.
**(3)** The artificial vessel has a compliance approximate to that of a vital vessel.

In case of the artificial vessel reinforced with the tubular material made of fiber, the vessel has a high strength and durability and a stress-strain curve of the vessel can be approximated to that of a vital vessel. In case of the artificial vessel reinforced with the heat-set tubular material made of fiber, the vessel can be subjected to the sterilization procedure by boiling or by high-pressure steam.

In addition, the artificial vessel of the present invention has the following useful properties, since the vessel wall of the artificial vessel is made of the elastomer having the porous structure and the vessel can be reinforced, as the occasion demands, with the tubular material made of fiber.

**(4)** A surgical needle easily penetrates to the artificial vessel, and thus the vessel is easily sutured.
**(5)** A bore formed by a needle can be closed in itself.

(6) A kinking cannot be formed in the practical use where blood pressure is applied.
(7) The artificial vessel has a high durability.

The artificial vessel of the present invention having the above-mentioned properties has an excellent patency and a good operatability in the vascular reconstructive surgery.

Therefore, the artificial vessel of the present invention can be used as an artificial vessel, an artificial vessel for by-pass, a material for patch, in the vascular reconstructive surgery of a vital vessel, moreover, a blood access. Especially, the artificial vessel of the present invention is preferably used as an artificial vessel for artery having a compliance of from 0.l to 0.8. Also, the artificial vessel of the present invention can be used as an artificial vessel of small-caliber for artery having the inner diameter of from about l to about 6 mm and a compliance of from 0.l to 0.5, which has not hitherto been available in clinical use. Thus, the artificial vessel of the present invention is preferably used for the artificial vessel in vascular reconstructive surgery of arteries below knees and for the artificial vessel for by-pass between aorta and coronary. In addition, the artificial vessel of the present invention can also be used as an artificial tube for a soft vital tube such as an urter.

The present invention is more particularly described and explained by means of the following Examples. It is to be understood that the present invention is not limited to the Examples and various changes and modifications may be made without departing from the scope of the present invention.

Example l

After synthesizing a pre-polymer with 27.35 parts (part by weight, hereinafter the same) of 4,4'-diphenylmethane diisocyanate and 54.7 parts of polyoxytetramethylene glycol (molecular weight: 2000), the pre-polymer chain was extended with 4.75 parts of ethylene glycol and l3.2 parts of polydimethylsiloxane having polyethylene glycol at the both ends (average molecular weight of polyethylene glycol at the both ends: 68l, average molecular weight of polydimethylsiloxane: l040) to give a segmented polyurethane containing polydimethylsiloxane in the main chain.

The thus obtained polyurethane had a tensile strength of 350 kg/cm$^2$, an elongation of 670 % and a critical surface tension calculated from Zisman plot of 28 dyn/cm.

Into a mixed solvent of 60 ml of dioxane and 30 ml of N,N-dimethylacetamide, l5 g of casein having a particle size of not more than 30 μm was dispersed with stirring by a homogenizer and thereto l0 g of the above polyurethane was added to dissolve with stirring. A glass lod having a diameter of 3 mm was immersed into the dispersion liquid and then taken out to coat the dispersion liquid on the glass lod, which then immersed into ethylene glycol to deposit the elastomer.

The porosity percentage at the thickness of about l00 μm from the inner surface of the artificial vessel was measured at this stage as will be mentioned hereinafter.

The above procedure was further repeated three times employing l0 g of casein and the same dispersion liquid as above. After sufficiently depositing the elastomer, the obtained material was washed with water and the glass lod was pull out to give a tubular material, which was immersed into an aqueous solution of sodium hydroxide at pH l3.5 to remove casein with extraction and then washed with water sufficiently to give an artificial vessel.

The obtained artificial vessel had an inner diameter of about 3 mm and an outer diameter of about 4.l mm. From an observation with a scanning type electron microscope, there were many circular to oval openings of pores having a diameter of about 20 μm on the inner surface of the vessel wall and openings of pores with a round to indefinite shape having a diameter of from about l to about l0 μm on the outer surface of the vessel wall. A cross section of the vessel wall had a network structure.

By calculating the porosity percentage of the artificial vessel from the equation (l), it proved that the artificial vessel had the porosity percentage of about 88 % at the thickness of about l00 μm from the inner surface and of 84 % as a whole.

The artificial vessel proved to have the porosity by passing water through the vessel wall at a pressure of l20 mmHg, about 50 ml/min. of water penetrating to the outside per l cm$^2$ of the inner surface.

After pre-clotting blood of bovine in the vessel and cutting the pre-clotted vessel to 8 cm, the artificial vessel was inserted into a closed circuit. The ACD blood of bovine was fed into the closed circuit by a quantitative pump which feed 0.05 ml per stroke, and the change of the inner pressure was measured. The compliance proved to be 0.4 from calculation according to the equation (2) on the basis of the number of strokes and the chage of the inner pressure.

The artificial vessel of about 7 cm length was grafted to a femoral artery of an adult mongrel dog. The grafted vessel showed a patency for not less than two months.

The artificial vessel did not fray when cutting at any point, and was excellent in suturing property. In addition, the bores of the surgical needle were closed in themselves when the needle was removed. Further, the vessel tended not to form a kinking under an inner pressure of from 50 to l50 mmHg.

From th above obtained results, it is clear that the artificial vessel has excellent properties as an artificial vessel of small-caliber for artery.

Example 2

Before the last procedure of immersing the glass lod into the dispersion liquid in Example I, a tubular material having a diameter of about 4 mm prepared by knitting covered yarn, which was prepared by winding tetron fiber of 50 deniers on spandex of I0 deniers, with a ribbon knitting machine of 24 needle was covered on the glass lod coated with the deposited elastomer. The procedure in Example I was repeated other than the above procedure to prepare an artificial vessel reinforced with the tubular material made of fiber.

The obtained artificial vessel had an inner diameter of 3 mm and an outer diameter of about 4.5 mm. The porosity percentage calculated from the equation (I) was 88 % at the thickness of about I00 μm from the inner surface, and 85 % as a whole.

The obtained artificial vessel was observed as in Example I to prove that it had the same contact surface with blood as the artificial vessel prepared in Example I. A compliance and a penetration volume of water measured as in Example I were 0.3 and about 40 ml, respectively.

By using Shimazu Autograph AG 2000A, a stress-strain curve was measured. The artificial vessel had a stress-strain curve approximate to that of a vital vessel.

A patency of the artificial vessel was measured as in Example I and proved to be not less than two months.

Example 3

Into a mixed solvent of 55 ml of N,N-dimethylacetamide and 30 ml of propylene glycol, I5 g of polyurethane as in Example I was dissolved with stirring at 70°C. The solution had a cloud point of about 40°C.

A glass lod having a diameter of 3 mm was immersed into the above solution at 70°C and then taken out to coat the glass lod with the solution uniformly. The coated glass lod was allowed to stand in air until the coating layer became cloudy and the glass lod was immersed into water at I5°C to sufficiently replace the mixed solvent with water. The above procedure was further repeated two times. After sufficient washing with water, the glass lod was pull out to give an artificial vessel.

The obtained artificial vessel had an inner diameter of about 3 mm and an outer diameter of about 4 mm. By the observation with a scanning type electron microscope, oval openings of pores having a maximum diameter of about I0 μm were present almost uniformly on the inner surface of the vessel wall. A cross section of the vessel wall had a network structure. A porosity percentage, a porosity, and a compliance were measured as in Example I and proved to be 80 %, I0 ml/cm$^2$•min and 0.35, respectively.

A patency of the artificial vessel was measured as in Example I and proved to be not less than two months.

Example 4

A dispersion liquid was prepared by adding II7 ml of N,N-dimethylacetamide and II7 ml of dioxane to a mixture of 32 g of polyurethane as in Example I and 48 g of casein having a particle size of from 30 μm to 40 μm with stirring. A glass lod was immersed into the dispersion liquid and then taken out to coat the dispersion liquid on the glass lod uniformly. The coated glass lod was immersed into an aqueous solution containing 30 % by volume of dioxane to deposit the elastomer.

The above procedure was repeated once more using the same dispersion liquid as above excepting each 27 g of polyurethane and of casein.

A tubular material prepared by knitting Woolie polyester fiber of 50 deniers with a ribbon knitting machine of 24 needle was sterilized by high-pressure steam at I2I°C for 20 minutes and then dried. The thus obtained tubular material having a diameter of about 4 mm was covered on the glass lod having the deposited elastomer. The glass lod covered with the tubular material was immersed into the same dispersion liquid as above excepting each 8.5 g of polyurethane and of casein, and then taken out to deposit the elastomer. Thereafter, the procedure as in Example I was repeated to give an artificial vessel.

The obtained artificial vessel had an inner diameter of 3 mm and an outer diameter of about 4.2 mm. The porosity percentage was 87 % at the thickness of about 300 μm from the inner surface, and 84 % as a whole.

A compliance and a penetration volume measured as in Example I were 0.45 and about 70 ml/cm$^2$, respectively. A stress-strain curve measured with Shimazu Autograph AG-2000A was shown in Fig. I, which was approximate to that of a vital vessel.

The artificial vessel was sterilized by high-pressure steam at I2I°C for 20 minutes without any change in shape or size, which showed that the artificial vessel can be subjected to the sterilization procedure by high-pressure steam.

A patency of the artificcial vessel was measured as in Example I and proved to be not less than two months.

## Claims

1. An artificial vessel, wherein the vessel wall is made of a thermoplastic elastomer having a porous structure and the vessel has a porosity percentage of from 95 to 75%.

2. The artificial vessel of Claim 11, wherein the porosity percentage at the thickness of at least 30 µm from the inner surface of the vessel wall is from 95 to 80%, and the porosity percentage at the rest of the thickness of at least 30 µm from the inner surface of the vessel wall.

3. The artificial vessel of Claim 1, wherein the vessel is reinforced with tubular material made of fiber.

4. The artificial vessel of Claim 1, wherein the vessel is reinforced with heat-set tubular material made of fiber.

## Patentansprüche

1. Künstliches Gefäß, worin die Gefäßwand aus einem thermoplastischen Elastomer mit einer porösen Struktur hergestellt ist und das Gefäß einen Porositätsanteil von 95 bis 75% hat.

2. Künstliches Gefäß nach Anspruch 1, worin der Porositätsanteil bei der Dicke von mindestens 30, µm von der inneren Oberfläche der Gefäßwand von 95 bis 80% ist, und der Porositätsanteil im Rest der Dicke ab mindestens 30, µm von der inneren Oberfläche der Gefäßwand kleiner ist als der bei der Dicke von mindestens 30, µm von der inneren Oberfläche der Gefäßwand.

3. Künstliches Gefäß nach Anspruch 1, worin das Gefäß mit röhrenförmigem, aus Faser hergestelltem Material verstärkt ist.

4. Künstliches Gefäß nach Anspruch 1, worin das Gefäß mit thermofixiertem röhrenförmigem, aus Faser hergestelltem Material verstärkt ist.

## Revendications

1. Vaisseau artificiel, dans lequel la paroi du vaisseau est faite d'un élastomère thermoplastique ayant une structure poreuse et le vaisseau possède un pourcentage de porosité compris entre 95 et 75%.

2. Vaisseau artificiel selon la revendication 1, dans lequel le pourcentage de porosité sur l'épaisseur d'au moins 30 µm à partir de la surface interne de la paroi du vaisseau est compris entre 95 et 80%, le pourcentage de porosité sur ce qui reste de l'épaisseur d'au moins 30 µm à partir de la surface interne de la paroi du vaisseau est inférieur à celui sur l'épaisseur d'au moins 30 µm à partir de la surface interne de la paroi du vaisseau.

3. Vaisseau artificiel selon la revendication 1, dans lequel le vaisseau est renforcé par un matériau tubulaire fait de fibres.

4. Vaisseau artificiel selon la revendication 1, dans lequel le vaisseau est renforcé par un matériau tubulaire, fixé à la chaleur, fait de fibres.

FIG. 1